# FASCICULE DE BREVET EUROPEEN

(11) **EP 2 429 379 B1**
(45) Date de publication et mention de la délivrance du brevet: **31.08.2016**
(21) Numéro de dépôt: 10727777.4
(22) Date de dépôt: 07.05.2010
(51) Int. Cl.: G06K 9/00, A61B 3/113

(54) **PAIRE DE LUNETTES OPHTALMIQUES ADAPTEE POUR CARACTERISER UNE DIRECTION DE REGARD D'UN PORTEUR**
OPHTHALMISCHE BRILLE ZUR CHARAKTERISIERUNG DER BLICKRICHTUNG EINES TRÄGERS
OPHTHALMIC SPECTACLES FOR CHARACTERIZING THE DIRECTION OF GAZE OF A WEARER

(30) Priorité: 12.05.2009 FR 0953127
(43) Date de publication de la demande: 21.03.2012
(73) Titulaire: ESSILOR INTERNATIONAL (COMPAGNIE GENERALE D'OPTIQUE), 94220 Charenton-Le-Pont (FR)
(72) Inventeur: BONNIN, Thierry, F-94220 Charenton Le Pont (FR); BROSSIER, Thibault, F-94220 Charenton Le Pont (FR); ROUSSEAU, Benjamin, F-94220 Charenton Le Pont (FR)
(74) Mandataire: Cabinet Plasseraud
(86) Numéro de dépôt international: PCT/FR2010/050895
(87) Numéro de publication internationale: WO 2010/130932

(56) Documents cités:
- EP-A1- 0 733 338
- WO-A1-99/18868
- US-A- 3 689 135
- US-A- 4 300 818
- US-A- 5 070 883

## Description

La présente invention concerne une paire de lunettes ophtalmiques qui est adaptée pour caractériser une direction de regard d'un porteur de cette paire de lunettes.

De nombreux systèmes existent déjà, qui permettent de détecter les mouvements oculaires d'un sujet en temps réel. Par exemple, le document US 5,966,197 décrit un système de chirurgie oculaire par ablation laser, qui permet de détecter et de compenser les mouvements oculaires d'un patient. Ce système forme une image de l'oeil, et la position de celui-ci en rotation dans l'orbite est repérée en identifiant la position du limbe dans l'image. On rappelle que le limbe est la limite entre la sclérotique et l'iris de l'oeil, et qu'il peut être facilement détecté dans une image de l'oeil en lumière du domaine proche infrarouge, pour des longueurs d'onde qui sont comprises entre 900 nm (nanomètre) et 1200 nm. En effet, la sclérotique possède un coefficient d'intensité de réflexion lumineuse qui est élevé, de l'ordre de 90%, dans ce domaine spectral, alors que l'iris possède un coefficient d'intensité de réflexion plus faible, de l'ordre de 40%.

Le document US 4,300,818 qui est considéré comme représentant l'état de la technique le plus proche quant au sujet des revendications 1 et 11 décrit un système de paire de lunettes opthalmiques comprenant une monture et deux verres maintenus dans la monture pour être situés respectivement devant les yeux d'un porteur des dites lunettes, et permettant une vision distincte pour le porteur à travers chaque verre

D'autres systèmes de détection des mouvements oculaires existent aussi, qui sont destinés à être utilisés par des patients en état conscient. Certains de ces autres systèmes sont constitués d'appareils à placer sur la tête du sujet, et forment des images des deux yeux du sujet en même temps que celui-ci regarde des objets de son environnement. Mais bien qu'un sujet qui est équipé d'un de ces appareils conserve sa mobilité, il ne s'agit pas de dispositifs qui soient utilisables dans la vie courante, mais de dispositifs dont l'utilisation est limitée à des séances de mesures pratiquées sur le sujet.

Or certaines applications nécessitent des systèmes de détection des mouvements oculaires, qui soient compatibles avec une utilisation nomade. Il existe donc un besoin pour un système de détection des mouvements oculaires qui soit à la fois léger et esthétique, et qui ne provoque pas de gêne pour le sujet lors d'une utilisation prolongée dans la vie courante. En outre, un tel système est de préférence peu onéreux, pour permettre d'équiper un grand nombre d'individus. Mais il n'existe pas à ce jour de systèmes de détection des mouvements oculaires qui réponde à ces besoins de façon satisfaisante.

Un but de la présente invention est donc de fournir un système de détection des mouvements oculaires qui soit compatible avec une utilisation nomade dans la vie courante.

Un autre but de l'invention est de fournir une caractérisation en temps réel de la direction d'un objet qui est observé par un utilisateur du système de détection des mouvements oculaires.

Un autre but encore de l'invention est de fournir une caractérisation de la direction de regard de l'utilisateur, et de ses mouvements oculaires qui déterminent les variations de cette direction de regard.

Pour cela, l'invention propose une paire de lunettes ophtalmiques qui comprend une monture et deux verres maintenus dans la monture pour être situés respectivement devant les yeux d'un porteur des lunettes, et qui permet une vision distincte pour le porteur à travers chaque verre, la paire de lunettes comprenant pour chaque verre :
- au moins une source d'un rayonnement pouvant être un rayonnement infrarouge, qui est sélectionnée pour que ce rayonnement soit réfléchi par la sclérotique et par l'iris de chaque oeil du porteur conformément à des coefficients d'intensité de réflexion différents respectivement pour la sclérotique et pour l'iris ;
- au moins un détecteur, qui est disposé pour mesurer des intensités respectives de parties du rayonnement produit par la source, réfléchies respectivement dans des zones oculaires dans chacune desquelles se déplace une portion différente d'une limite entre la sclérotique et l'iris de l'oeil situé derrière le verre lors de mouvements oculaires ; et
- au moins quatre sections de sortie ou d'entrée de rayonnement qui sont disposées dans le verre, chaque section de sortie étant disposée pour diriger une partie du rayonnement produit par la source vers au moins une des zones oculaires, et chaque section d'entrée étant disposée pour collecter une partie du rayonnement qui est réfléchie dans l'une de ces zones oculaires.

Selon une première caractéristique de l'invention, ces source(s), détecteur(s) et sections de sortie et d'entrée de rayonnement sont en outre disposés de façon à former au moins deux chemins de rayonnement qui comportent chacun une réflexion sur l'oeil du porteur situé derrière le verre, dans l'une des zones oculaires où se déplacent les portions de la limite entre la sclérotique et l'iris de l'oeil. La paire de lunettes comprend alors des moyens pour séparer les parties de rayonnement qui sont transmises entre ladite au moins une source de rayonnement et ledit au moins un détecteur respectivement par des chemins de rayonnement différents.

Selon une seconde caractéristique de l'invention, la paire de lunettes comprend aussi une unité de calcul qui est adaptée pour caractériser une direction de regard du porteur vers un point d'observation, en fonction des intensités qui sont mesurées simultanément pour les deux verres.

Selon une troisième caractéristique de l'invention, la paire de lunettes comprend autant de sections de sortie de rayonnement que de sections d'entrée de rayonnement pour chaque verre. Chacune de ces sections de sortie est juxtaposée dans le verre avec l'une des sections d'entrée pour former avec elle un couple séparé d'émission-réception de rayonnement, et chaque couple d'émission-réception est disposé devant une des zones oculaires où se déplace l'une des portions de la limite entre la sclérotique et l'iris de l'oeil du porteur qui est situé derrière ce verre lors des mouvements oculaires.

Ainsi, la présente invention fournit un système de détection des mouvements oculaires d'un sujet, qui se présente sous forme d'une paire de lunettes, avec des sections de sortie et d'entrée de rayonnement qui sont intégrées dans les verres. Le système est donc léger et produit un encombrement très réduit, grâce à sa présentation sous forme d'une paire de lunettes. Il peut donc être utilisé dans la vie courante, notamment lorsque l'utilisateur se déplace. En particulier, un porteur qui est équipé d'une paire de lunettes selon l'invention conserve une liberté de mouvement totale.

En outre, étant donné que les sections de sortie et d'entrée de rayonnement sont intégrées dans chaque verre, la paire de lunettes est dépourvue de tout réflecteur additionnel et de tout système de prise de vue dirigé vers les yeux du porteur, qui serait situé en avant de son visage en plus des verres de lunettes. Un système de détection de mouvements oculaires selon l'invention est donc esthétique sur le visage du porteur, et ne provoque aucune gêne visuelle pour celui-ci.

En outre, la détection des mouvements oculaires qui est réalisée en utilisant une paire de lunettes selon l'invention est fondée sur la détection des positions de portions différentes du limbe de chaque oeil. Pour cela, au moins deux faisceaux de rayonnement sont dirigés à partir de chaque verre vers les zones de déplacement des portions du limbe de l'oeil correspondant du porteur, et les intensités respectives de ces faisceaux après réflexion sur chaque oeil dans ces zones sont mesurées. Chaque faisceau possède un chemin de rayonnement différent. Son intensité après réflexion, lorsqu'il traverse en retour l'une des sections d'entrée du verre, varie en fonction de la position en rotation des yeux, puisque chaque faisceau est plus ou moins réfléchi selon qu'il atteint la surface de l'oeil plutôt dans la sclérotique ou plutôt dans l'iris. La direction de regard du porteur vers un objet qui est regardé par celui-ci à un instant donné est alors déduite d'une combinaison des positions des limbes des deux yeux. De cette façon, la direction de regard du porteur peut être déterminée quelque soit la convergence de ses yeux, cette convergence étant provoquée par le rapprochement ou l'éloignement de l'objet qui est regardé par le porteur.

Enfin, la répartition des sections de sortie et d'entrée de rayonnement à l'intérieur du verre, selon des couples d'émission-réception qui sont formés chacun d'une section de sortie et d'une section d'entrée dédiées à ce couple, permet de collecter sans mélange les rayonnements qui sont transmis par des chemins distincts. La direction de regard du porteur est alors caractérisée avec une précision supérieure. Une telle configuration est particulièrement adaptée lorsque chaque section de sortie dirige la partie de rayonnement correspondante à l'intérieur d'un cône d'émission qui est étroit. Etant donné que les réflexions du rayonnement sur l'oeil sont principalement spéculaires, chaque section d'entrée ne collecte que la partie réfléchie du rayonnement qui a été transmis par la section de sortie correspondante, appartenant au même couple d'émission-réception que la section d'entrée. Ainsi, grâce à l'invention, les mesures optiques qui sont effectuées pour des chemins de rayonnement qui sont différents sont elles-mêmes indépendantes.

Lorsque les sections de sortie ou d'entrée de rayonnement de chaque verre ne forment que deux chemins de rayonnement, une seule coordonnée angulaire de la direction de regard peut en général être déterminée, par exemple la hauteur de la direction de regard ou son azimut.

Dans des réalisations préférées de l'invention, les dits au moins une source, au moins un détecteur et au moins quatre zones de sortie ou d'entrée de rayonnement de chaque verre sont disposés pour former au moins trois chemins distincts pour le rayonnement, avec des réflexions respectives qui sont réparties angulairement autour d'une direction centrale d'une orbite de chaque oeil. La direction de regard du porteur peut alors être déterminée entièrement, notamment par les deux angles de hauteur et d'azimut pour chaque instant de mesure. Pour de telles réalisations préférées, chaque verre comporte au moins quatre sections de sortie ou d'entrée de rayonnement.

Dans divers modes de réalisation de l'invention, les dits au moins une source, au moins un détecteur et au moins quatre zones de sortie ou d'entrée de rayonnement peuvent être disposés, pour chaque verre, de façon à former au moins deux paires de chemins distincts pour le rayonnement.

Selon un premier mode de réalisation de l'invention, chaque chemin peut avoir une réflexion dans une zone oculaire où se déplace une portion droite ou gauche de la limite entre la sclérotique et l'iris de l'oeil du porteur qui est situé derrière le verre correspondant. Ces zones oculaires des chemins de chaque paire peuvent être situées à une même hauteur de zones oculaires qui est associée à cette paire. En outre, les deux paires de chemins sont associées à des hauteurs respectives qui sont distinctes et communes aux deux verres. Dans ce cas, l'unité de calcul peut éventuellement être adaptée pour caractériser la direction de regard du porteur en fonction des intensités qui sont mesurées simultanément pour les parties de rayonnement transmises par les chemins associés à une même des hauteurs de zones oculaires. Cette hauteur qui est sélectionnée par l'unité de calcul est alors la même pour les deux verres, pour caractériser en azimut la direction de regard du porteur à un instant donné.

Selon un second mode de réalisation de l'invention, chaque chemin d'une première des paires de chemins peut avoir une réflexion dans une zone oculaire où se déplace une portion droite ou gauche de la limite entre la sclérotique et l'iris de l'oeil du porteur qui est situé derrière le verre. Simultanément, chaque chemin d'une seconde des paires de chemins peut avoir une réflexion dans une zone oculaire où se déplace une portion supérieure ou inférieure de la limite entre la sclérotique et l'iris de l'oeil du porteur. Ce second mode de réalisation de l'invention permet de déterminer la direction de regard du porteur, d'une façon qui est équivalente pour une rotation de cette direction vers l'un des côtés droit ou gauche du porteur d'une part, et pour une inclinaison de la direction de regard vers le haut ou le bas du champ de vision de vision du porteur d'autre part.

Dans divers modes de réalisation de l'invention, certains des perfectionnements suivants peuvent être utilisés séparément ou en combinaison :
- deux premières sections de sortie de rayonnement peuvent être disposées dans chaque verre à une même première hauteur, et deux secondes sections de sortie de rayonnement peuvent être disposées dans chaque verre à une même seconde hauteur différente de la première hauteur ; et
- pour chaque verre, certaines au moins des sections de sortie de rayonnement peuvent être disposées sous forme de deux colonnes qui sont situées respectivement dans des parties droite et gauche de ce verre, avec chaque section de sortie de la colonne qui est située dans la partie droite du verre appartenant à un chemin de rayonnement qui comporte une réflexion sur l'oeil du porteur dans la zone oculaire où se déplace la portion droite de la limite entre la sclérotique et l'iris de cet oeil, et chaque section de sortie de la colonne qui est située dans la partie gauche du verre appartenant à un chemin de rayonnement qui comporte une réflexion sur l'oeil du porteur dans la zone oculaire où se déplace la portion gauche de la limite entre la sclérotique et l'iris de l'oeil.

Eventuellement, une paire de lunettes selon l'invention peut comprendre en outre des moyens pour varier une caractéristique de l'un au moins des verres, en fonction de la direction de regard du porteur qui est caractérisée par l'unité de calcul. En particulier, les moyens pour varier la caractéristique de l'un des verres peuvent être compris dans ce verre.

Une paire de lunettes selon l'invention peut être utilisée avantageusement pour l'une des applications suivantes, qui sont citées à titre d'exemples :
- en orthoptie, lors de séances de rééducation visuelle. En effet, la paire de lunettes permet de soumettre un patient qui est équipé de celle-ci à des exercices de rééducation plus adaptés et plus contrôlés ;
- pour un sujet qui est atteint de dégénérescence maculaire. En effet, il est possible d'occulter dynamiquement une partie de son champ de vision en fonction de la direction de son regard, afin de favoriser sa perception visuelle dans la zone rétinienne périphérique ;
- pour un sujet tétraplégique. En effet, une paire de lunettes selon l'invention peut constituer une interface d'entrée de communication, qui permet au sujet de transmettre des données à un outil de saisie informatique, en variant volontairement la direction de son regard ;
- de caractériser des mouvements oculaires involontaires chez un sujet, tels que des saccades de la direction de son regard, par exemple pour identifier un état de stress psychologique de ce sujet ;
- pour effectuer un traitement d'image qui soit asservi à la direction de regard du porteur de la paire de lunettes ; et
- pour présenter des données visuelles au porteur de la paire de lunettes, d'une façon qui soit adaptée dynamiquement en fonction de sa direction de regard à un instant donné. La présentation des données visuelles peut alors apparaître au porteur en superposition avec sa vision naturelle, et être asservie en fonction des variations de sa direction de regard pour cette vision naturelle.

L'invention propose aussi un verre de lunettes qui est adapté pour être assemblé dans une paire de lunettes telle que décrite précédemment. Un tel verre comprend :
- au moins une source d'un rayonnement pouvant être un rayonnement infrarouge, chaque source étant intégrée au verre et sélectionnée pour que le rayonnement soit réfléchi par la sclérotique et par l'iris d'un oeil d'un porteur du verre, conformément à des coefficients d'intensité de réflexion différents respectivement pour la sclérotique et pour l'iris ;
- au moins un détecteur de ce rayonnement, chaque détecteur étant intégré au verre et disposé pour mesurer une intensité d'une partie du rayonnement produit par l'une des sources, qui est réfléchie par l'oeil dans une zone oculaire dans laquelle se déplace une portion différente d'une limite entre la sclérotique et l'iris de l'oeil lors de mouvements oculaires ;
- des pistes conductrices transparentes, qui sont intégrées au verre et qui relient électriquement des bornes de chaque détecteur et de chaque source, et qui sont dirigées radialement dans une partie périphérique du verre ; et
- au moins quatre sections de sortie ou d'entrée de rayonnement qui sont disposées dans le verre pour former des chemins de rayonnement avec l'oeil d'un porteur de ce verre de lunettes comme décrit précédemment.

La disposition radiale des pistes dans la partie périphérique du verre permet notamment de prévoir des contacts électriques dans la monture à des emplacements déterminés, pour relier électriquement les pistes du verre à d'autres composants électroniques portés par la monture. En outre, cette disposition radiale permet que le verre soit détouré selon la forme de son logement dans la monture, quelque soit cette forme, en conservant la possibilité de prévoir les emplacements des contacts électriques de la monture.

D'autres particularités et avantages de la présente invention apparaîtront dans la description ci-après d'exemples de réalisation non limitatifs, en référence aux dessins annexés, dans lesquels :
- les figures 1a et 1b sont des vues en perspective et en plan, respectivement, montrant l'utilisation d'une paire de lunettes selon un premier mode de réalisation de l'invention ;
- les figures 2a et 2b sont des vues agrandies de face et en plan, respectivement, illustrant le premier mode de réalisation de l'invention ;
- les figures 2c et 2d correspondent à la figure 2b, respectivement pour deux positions différentes d'un oeil d'un porteur de la paire de lunettes ;
- la figure 3 correspond à la figure 2a pour un deuxième mode de réalisation de l'invention ;
- les figures 4a et 4b sont des vues respectives en plan et en section transversale d'un verre selon l'invention ;
- la figure 5 illustre un type différent de mode de réalisation de l'invention ;
- les figures 6a à 6d correspondent respectivement aux figures 2a à 2d pour un troisième mode de réalisation de l'invention ; et
- la figure 7 correspond à la figure 3 pour un quatrième mode de réalisation de l'invention.

Pour raison de clarté, les dimensions des éléments qui sont représentés dans ces figures ne sont pas en proportion avec des dimensions réelles, ni avec des rapports de dimensions réels. En outre, des références identiques sur des figures différentes désignent des éléments identiques ou qui ont des fonctions identiques.

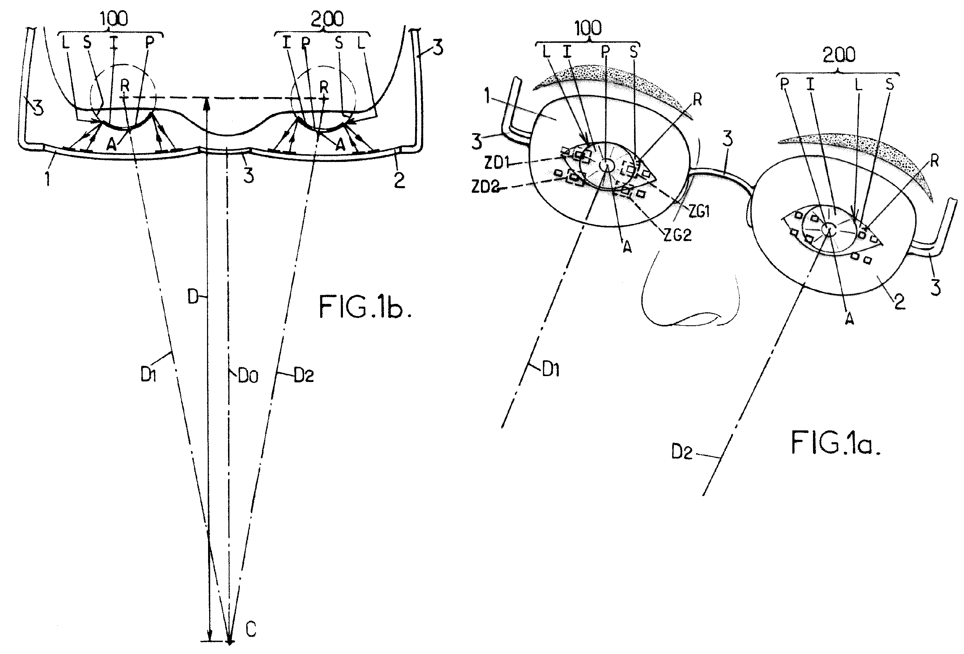

Conformément aux figures 1a et 1b, une paires de lunettes comprend une monture 3 et deux verres ophtalmiques, qui sont référencés 1 et 2 pour le verre droit et le verre gauche, respectivement. La monture 3 maintient les verres 1 et 2 dans des positions relatives qui sont fixes, et permet de les placer devant les yeux d'un porteur de la paire de lunettes d'une façon qui est constante lors de durées de port successives. Les verres 1 et 2 peuvent être assemblés définitivement dans la monture 3, en utilisant l'un des procédés d'assemblage connus des opticiens. Alternativement, les verres 1 et 2 peuvent être ajoutés par l'invention à une paire de lunettes initiale qui comprend la monture 3. La paire de lunettes initiale peut être une paire de lunettes de protection solaire et/ou de correction ophtalmique. Dans ce cas, les verres 1 et 2 peuvent être maintenus sur la paire de lunettes initiale de façon amovible, par exemple par clipsage.

Les références 100 et 200 désignent les yeux du porteur, respectivement son oeil droit et son oeil gauche. Pour chaque oeil 100, 200 du porteur, les références S, I, P, L et R désignent la sclérotique, l'iris, la pupille, le limbe et le centre de rotation de l'oeil. De façon connue, l'iris I est une couronne circulaire dont le diamètre interne est variable et détermine la taille de la pupille P, et dont le diamètre externe est constant. Le limbe L est la limite externe de l'iris I, entre cet iris et la sclérotique S. C'est donc un cercle de taille constante qui est fixe par rapport à l'oeil correspondant lorsque ce dernier tourne autour de son centre de rotation R. Visuellement, le limbe L est la limite circulaire entre la sclérotique S qui est blanche et l'iris coloré I.

Pour chaque oeil 100, respectivement 200, l'axe D1, resp. D2, qui passe par le centre de rotation R et le centre A de la pupille P correspondante est l'axe optique de cet oeil. Le centre A de la pupille P est aussi le sommet du cristallin, et est appelé «apex». L'axe optique D1, resp. D2, est fixe par rapport à l'oeil 100, resp. 200, de sorte qu'il est entraîné en rotation avec le limbe L. Les axes optiques D1 et D2 des yeux 100 et 200 convergent vers un point commun C (figure 1 b), qui est appelé point de convergence des yeux et qui est l'emplacement d'un objet regardé par le porteur à un instant donné. La direction moyenne D0 des axes optiques D1 et D2 est la direction de regard du porteur à cet instant. Usuellement, la direction de regard D0 relie un milieu du segment entre les deux centres de rotation oculaire R et le point de convergence C. La distance d'observation, qui est notée D sur la figure 1b, est l'éloignement du point de convergence C par rapport aux centres de rotation R.

La présente invention, qui est décrite maintenant dans des modes de réalisation particuliers, permet de déterminer la direction de regard D0 par rapport au visage du porteur. Dans ces modes de réalisation, cette direction de regard D0 est déterminée à partir d'une détection de la position en rotation de chaque oeil 100, 200 par rapport au verre correspondant 1, 2. Ainsi, chaque verre 1, 2 selon l'invention permet de déterminer la position angulaire de l'axe optique D1, D2 de l'oeil 100, 200 correspondant. La direction de regard D0 du porteur est alors déduite des positions respectives des deux axes optiques D1 et D2.

Pour repérer l'axe optique de chaque oeil, on utilise deux angles α et β, appelés respectivement hauteur et excentricité. La hauteur α est usuellement identique pour les deux yeux 100 et 200, et est l'angle entre chaque axe optique D1 ou D2 et un plan de référence qui est horizontal lorsque la tête du porteur est elle-même verticale. La valeur de hauteur de la direction de regard D0 est alors aussi égale à cette valeur commune.

L'excentricité β de l'axe optique D1 ou D2 de chaque oeil est l'angle entre cet axe et un plan médian du visage, qui est vertical lorsque la tête du porteur est elle-même verticale. L'excentricité β peut être comptée positivement vers le nez du porteur pour chaque oeil, et possède en général des valeurs absolues qui sont distinctes pour les deux yeux à un même instant. L'écart entre ces deux valeurs absolues détermine la convergence des yeux, c'est-à-dire la distance d'observation D. La valeur d'azimut de la direction de regard D0 est égale à la demi-différence des valeurs d'excentricité respectives des deux yeux, pour la convention d'orientation des angles d'excentricité qui vient d'être indiquée.

Pratiquement, la hauteur et l'excentricité de l'axe optique D1, D2 de chaque oeil 100, 200 est déterminée à partir de la position du limbe L de cet oeil. Plus précisément, des positions respectives de plusieurs portions du limbe L sont déterminées, en mesurant des intensités de rayonnements qui sont réfléchis par l'oeil dans des zones différentes de celui-ci. Pour cela, chaque verre est pourvu de sections d'émission du rayonnement, appelées sections de sortie, qui permettent de diriger un ou plusieurs faisceaux de rayonnement vers des zones de l'oeil, dites zones oculaires, dans lesquelles se déplacent les portions du limbe lorsque l'oeil tourne. Il est aussi pourvu de sections de collecte, appelées sections d'entrée, pour collecter des parties de ce rayonnement qui ont été réfléchies dans les zones oculaires. Les sections de sortie sont alimentées en rayonnement par au moins une source de rayonnement, et les sections d'entrée sont reliées optiquement à au moins un détecteur pour mesurer les intensités des parties du rayonnement qui sont collectées respectivement par ces sections d'entrée.

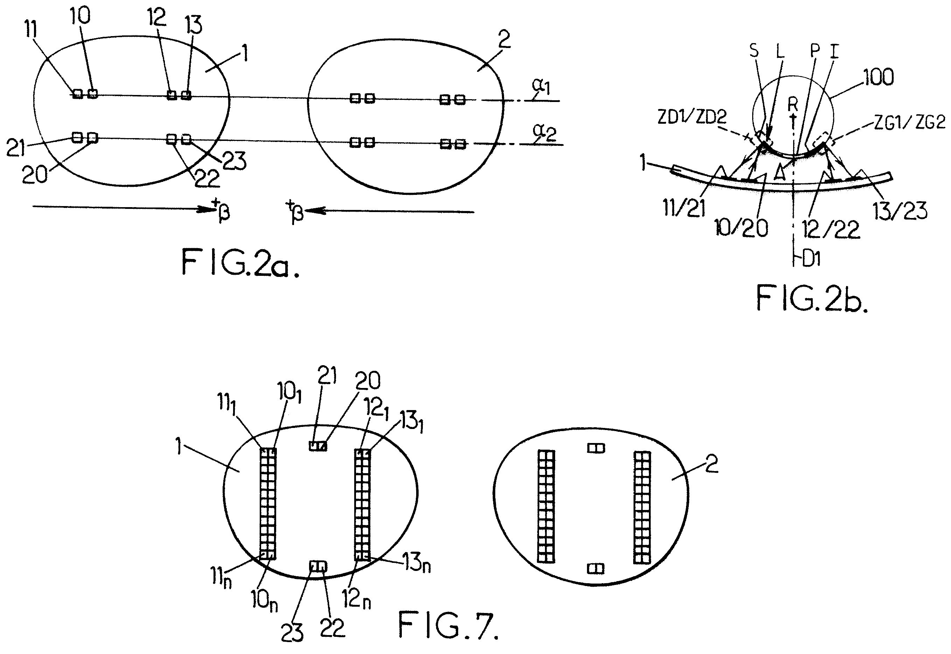

Sur le verre droit 1 des figures 2a à 2d, un premier couple d'émission-réception est constitué par la section de sortie 10 et par la section d'entrée 11. Les sections 10 et 11 sont juxtaposées, par exemple selon une direction horizontale, avec un décalage qui peut être compris entre 0,1 et 3 mm (millimètre). Un deuxième couple d'émission-réception est constitué par une autre section de sortie 12 et par une autre section d'entrée 13. Les couples 10/11 et 12/13 peuvent avoir des constitutions qui sont identiques. Ils peuvent être situés dans le verre 1 à des valeurs de la hauteur α qui sont égales, notées α₁. Ils sont situés en outre à des valeurs d'excentricité β qui sont différentes : le couple d'émission-réception 10/11 est situé en vis-à-vis de la zone latérale droite ZD1 de l'oeil où se déplace la portion droite du limbe L, et le couple d'émission-réception 12/13 est situé en vis-à-vis de la zone latérale gauche ZG1 de l'oeil où se déplace la portion gauche du limbe L. Chaque couple d'émission-réception détermine donc un chemin de rayonnement qui est indiqué sur les figures 2b à 2d, à partir de la section de sortie 10 ou 12 correspondante et jusqu'à la section d'entrée 11 ou 13 correspondante, et qui comprend un point de réflexion du rayonnement dans la zone ZD1 ou ZG1 correspondante. Ainsi, dans un tel mode de réalisation de l'invention, les différents chemins de rayonnement qui sont utilisés pour déterminer la position de l'oeil en rotation sont séparés par la façon de disposer les sections de sortie et d'entrée du rayonnement dans le verre.

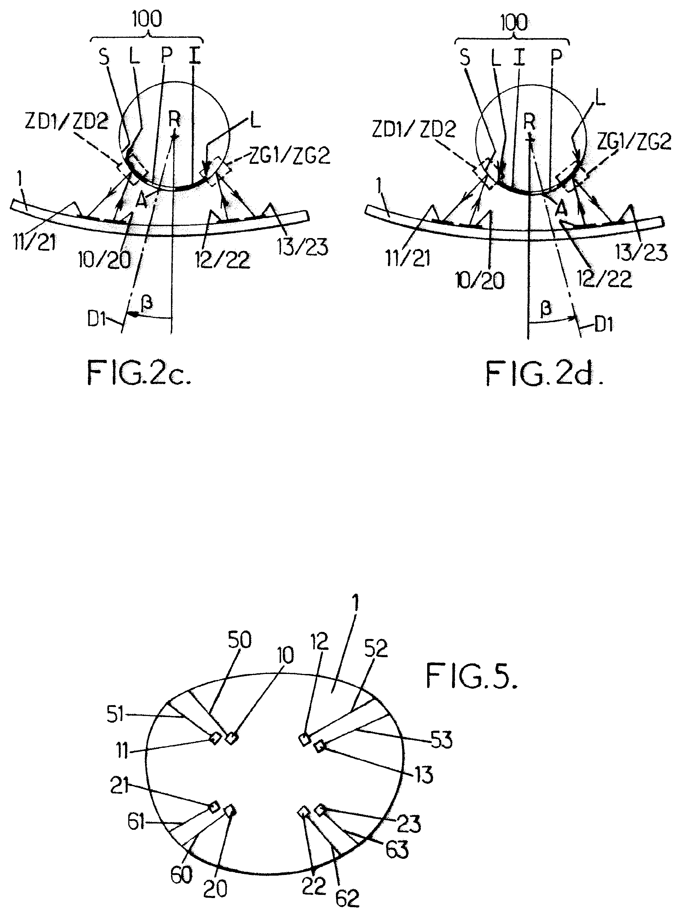

Les figures 2b, 2c et 2d représentent respectivement des positions en rotation de l'oeil droit 100, pour lesquelles l'axe optique D1 correspond à une valeur de l'excentricité β qui est nulle (figure 2b), négative ou orientée vers le côté temporal (figure 2c) et positive ou orientée vers le côté nasal (figure 2d). Lorsque le rayonnement utilisé possède une longueur d'onde qui est comprise entre 900 et 1200 nm, la différence entre l'intensité de réflexion de l'iris, qui est faible, et celle de la sclérotique, qui est plus élevée, permet de détecter la position de l'oeil 100. Ainsi, les réflexions qui sont détectées respectivement dans les zones latérales ZD1 et ZG1 par les couples d'émission-réception 10/11 et 12/13 ont des intensités qui sont sensiblement identiques dans le cas de la figure 2b, celle du couple 10/11 est inférieure à celle du 12/13 dans le cas de la figure 2c, et supérieure à cette dernière dans le cas de la figure 2d. Lorsque l'excentricité β de l'axe optique D1 varie continûment, les intensités mesurées pour les parties de rayonnement qui pénètrent respectivement par les sections d'entrée 11 et 13 varient aussi continûment, en sens opposés. Les couples d'émission-réception 10/11 et 12/13 permettent donc de déterminer la position angulaire de l'axe optique D1 de l'oeil 100, dans un plan horizontal lorsque la tête du porteur est verticale.

Eventuellement, le porteur peut être équipé de lentilles de contact qui améliorent la sensibilité de la détection de la position de chaque oeil. En effet, une telle lentille de contact peut recouvrir l'iris de l'oeil correspondant en restant centrée sur son axe optique, quelque soit la position en rotation de l'oeil. Elle peut alors être conçue pour adapter la valeur du coefficient d'intensité de réflexion apparent de l'iris, afin d'augmenter le contraste de réflexion entre l'iris et la sclérotique.

Pour cela, une unité calcul est associée à la paire de lunettes. Elle peut être incorporée dans l'une des branches de la monture 3, par exemple. Une telle unité de calcul est adaptée pour déterminer la position angulaire de l'axe optique D1 à partir d'intensités qui sont mesurées pour les parties de rayonnement réfléchies. Cette détermination de la position angulaire de l'axe optique D1 peut être effectuée par calcul. Alternativement, l'unité de calcul peut déterminer la position angulaire de l'axe optique D1 en lisant une table enregistrée qui indique cette position en fonction de valeurs d'intensité mesurées pour les différents chemins de rayonnement, servant d'entrées dans cette table.

Il est entendu que le verre gauche 2 présente une configuration qui est analogue à celle du verre droit 1, en étant symétrique de celui-ci par rapport au plan médian du visage du porteur. L'unité de calcul permet alors de déterminer la position angulaire de l'axe optique D2 de l'oeil gauche 200 du porteur, à des instants qui sont identiques à ceux des positions déterminées pour l'axe optique D1 de l'oeil droit 100. La direction du regard D0 est alors obtenue, ainsi que, éventuellement, la position du centre de convergence C des deux yeux et/ou la distance d'observation D.

Ainsi, comme il vient d'être décrit, l'unité de calcul peut être adaptée pour déterminer d'abord une position angulaire de l'axe optique de chaque oeil à partir des intensités qui sont mesurées simultanément pour le verre situé devant cet oeil, et pour les parties de rayonnement réfléchies dans certaines des zones oculaires où se déplacent les portions de la limite entre la sclérotique et l'iris de l'oeil. L'unité de calcul est alors aussi adaptée pour déterminer ensuite la direction de regard du porteur à partir des positions angulaires respectives des axes optiques des deux yeux qui ont été déterminées pour un même instant. Toutefois, un tel fonctionnement de l'unité de calcul en deux étapes successives n'est pas indispensable. La direction de regard D0 du porteur avec, éventuellement, la convergence de ses yeux et la distance d'observation D, peut (peuvent) être déduite(s) en une seule étape à partir d'une (de) combinaison(s) adéquate(s) des intensités qui sont mesurées simultanément pour les parties de rayonnement réfléchies dans les zones oculaires, où se déplacent les portions des limbes L correspondants.

Dans des premiers modes de réalisation de l'invention, chaque verre peut être prévu pour déterminer au moins deux paires de chemins de rayonnement, qui comprennent des réflexions sur l'oeil du porteur dans des zones latérales de celui-ci qui sont situées à une même première hauteur α₁ pour les chemins d'une même première paire, et à une même seconde hauteur α₂ pour les chemins d'une même seconde paire. Les figures 2a à 2d illustrent un tel mode de réalisation lorsque chaque chemin est déterminé par un couple d'émission-réception séparé de ceux des autres chemins. Ainsi, la section de sortie 10 avec la section d'entrée 11 d'une part, et la section de sortie 12 avec la section d'entrée 13 d'autre part, déterminent une première paire de chemins de rayonnement qui sont situés à la valeur de hauteur α₁. De même, la section de sortie 20 avec la section d'entrée 21 d'une part, et la section de sortie 22 avec la section d'entrée 23 d'autre part, déterminent une seconde paire de chemins de rayonnement qui sont situés à la valeur de hauteur α₂. Le couple d'émission-réception 20/21 détermine un chemin optique supplémentaire qui comprend un point de réflexion sur l'oeil 100 qui est situé dans une zone latérale droite ZD2 de celui-ci, à la hauteur α₂ en dessous de la zone ZD1. De même, le couple d'émission-réception 22/23 détermine un autre chemin optique supplémentaire qui comprend un point de réflexion sur l'oeil 100 qui est situé dans une zone latérale gauche ZG2 de celui-ci, à la hauteur α₂ en dessous de la zone ZG1. Dans ce cas, les hauteurs des zones oculaires qui sont associées aux deux paires de chemins de rayonnement peuvent avoir différence qui est comprise entre 10° et 45° en valeur absolue. Ainsi, l'une de ces valeurs de hauteur, par exemple α₁, peut correspondre à l'observation d'un objet qui est sensiblement situé à hauteur d'yeux pour le porteur. L'autre valeur de hauteur, α₂, peut correspondre à l'observation d'un objet qui est situé dans la partie inférieure du champ de vision du porteur. Lorsque la valeur d'azimut de la direction de regard D0 du porteur est seulement recherchée, l'unité de calcul peut alors être adaptée pour sélectionner l'une des valeurs α₁ ou α₂ de hauteur de zones oculaires pour caractériser cette valeur d'azimut. Une telle sélection peut être effectuée, notamment, en fonction d'une valeur d'un rapport signal/bruit de certaines au moins des intensités qui sont mesurées. De cette façon, les chemins de rayonnement qui peuvent être occultés par un abaissement des paupières du porteur ne sont pas pris en compte pour déterminer la direction de regard.

Une paire de lunettes qui est conforme au mode de réalisation des figures 2a à 2d peut aussi permettre de déterminer la hauteur α de la direction de regard D0 du porteur. En effet, la hauteur de l'axe optique D1, D2 de chaque oeil 100, 200 peut être déduite d'une comparaison, pour le verre qui est situé devant l'oeil correspondant, des intensités des rayonnements réfléchis qui sont détectés pour l'une et l'autre des valeurs de hauteur α₁ et α₂. Plus la direction de regard D0 est inclinée vers le bas, plus les portions latérales droite et gauche du limbe L apparaissent écartées, selon la direction horizontale, à la valeur de hauteur α₂, et resserrées à la valeur de hauteur α₁. Ainsi, la valeur de hauteur α de la direction de regard D0 peut être déduite d'une comparaison entre la somme des intensités des rayonnements réfléchis qui sont collectés par les sections d'entrée 21 et 23 d'une part, et la somme des intensités de rayonnements réfléchis qui sont collectés par les sections d'entrée 11 et 13 d'autre part.

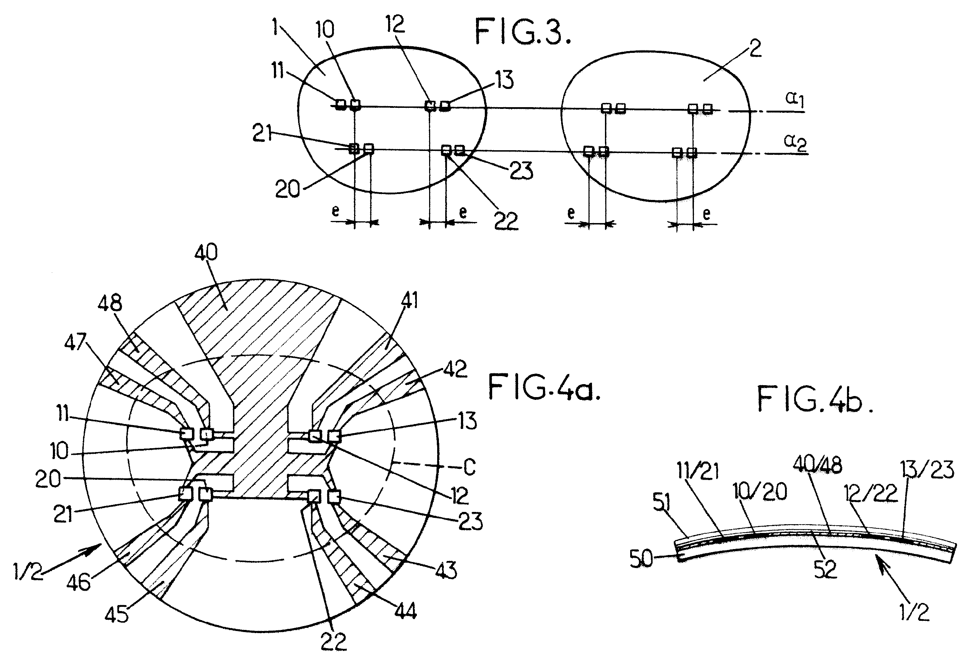

La figure 3 illustre un perfectionnement pour l'un des premiers modes de réalisation qui viennent d'être décrits, suivant lequel les couples d'émission-réception qui correspondent à l'une des valeurs de hauteur sont décalés latéralement par rapport aux couples d'émission-réception qui correspondent à l'autre valeur de hauteur. Ainsi, les couples d'émission-réception 20/21 et 22/23 qui définissent des réflexions sur l'oeil à la hauteur α₂ sont décalés de la distance e vers le côté nasal par rapport aux couples d'émission-réception 10/11 et 12/13 qui définissent des réflexions sur l'oeil à la hauteur α₁. Lorsque les verres 1 et 2 sont du type à addition progressive de puissance optique, couramment désignés par verres progressifs, les valeurs de hauteur α₁ et α₂ peuvent correspondre respectivement aux hauteurs dans chaque verre des directions de vision de loin et de près. Le décalage e peut alors être sensiblement égal au décalage qui résulte de la convergence variable des yeux entre les conditions de vision de loin et de près, et qui est couramment appelé inset. Dans ce cas, le décalage e peut être compris entre 4 et 6,5 mm. Plus généralement, il peut être compris entre 0 et 7 mm. Un tel décalage compense la variation de la convergence des yeux du porteur lorsque celui-ci abaisse la direction de son regard vers des objets qui sont de plus en plus proches, et peut simplifier la détermination de la direction de regard D0.

Selon un premier type de réalisations de l'invention, chaque source du rayonnement peut être une diode à émission lumineuse qui fonctionne à une longueur d'onde comprise entre 900 nm et 1200 nm. Dans ce cas, chaque section de sortie de rayonnement correspond à une portion de surface d'émission de cette diode. Alternativement, chaque source du rayonnement peut être une source VCSEL, pour «Vertical Cavity Surface Emitting Laser». De telles sources produisent chacune le rayonnement à l'intérieur d'un cône d'émission qui est étroit. Autrement dit, l'angle d'ouverture du faisceau de rayonnement qui est produit par chaque source est petit, usuellement inférieur à 10° (degré), voire inférieur à 5°. La section d'entrée du rayonnement qui appartient au même couple d'émission-réception qu'une des sections de sortie, est orientée pour collecter sélectivement la partie réfléchie du rayonnement qui est transmis par cette section de sortie. Cette sélectivité est obtenue grâce à la petite ouverture angulaire du faisceau de rayonnement transmis par la section de sortie, et grâce à l'orientation de la section d'entrée.

Parallèlement, chaque détecteur peut être une photodiode ou un phototransistor. Chaque section d'entrée de rayonnement correspond alors à une portion de surface photosensible de la photodiode ou du phototransistor.

Selon une combinaison particulièrement avantageuse, chaque source du rayonnement et chaque détecteur sont des composants micro-optoélectroniques à base de matériau semiconducteur, qui sont intégrés dans chaque verre. De cette façon, chaque verre est un élément autonome qui est adapté pour mettre en oeuvre l'invention, avec des coûts de fabrication et d'assemblage dans la monture qui peuvent être très faibles. Dans ce cas, chaque verre comprend en outre des pistes conductrices transparentes qui relient électriquement des bornes de chaque source et de chaque détecteur. Ces pistes relient les sources et détecteurs à l'unité de calcul qui peut être externe au verre de lunettes. De préférence, les pistes conductrices sont dirigées radialement dans une partie périphérique du verre. Grâce à une telle disposition des pistes, le verre peut être détouré selon un contour de son logement dans la monture 3, tout en repérant simplement les parties de ce contour dans lesquelles débouchent séparément les pistes conductrices. Par exemple, les pistes conductrices occupent des secteurs angulaires séparés dans la partie périphérique du verre. Conformément aux figures 4a et 4b, chaque verre 1, 2 peut comprendre une partie de base 50 et une partie d'encapsulation 51 qui sont solidaires, et chaque source du rayonnement, chaque détecteur et chaque piste conductrice peuvent être disposés entre les parties 50 et 51. Par exemple, la piste 40 peut constituer une borne de masse qui est commune à toutes les sources de rayonnement et détecteurs du verre, et les pistes 41 à 48 relient respectivement une autre borne de chacune des sources 10, 12, 20, 22 et de chacun des détecteurs 11, 13, 21, 23. Ces pistes, sources et détecteurs peuvent être formés sur la partie de base 50 et être enrobés dans une couche de colle transparente 52 qui assure la liaison mécanique avec la partie d'encapsulation 51. Les sources et détecteurs du rayonnement ont avantageusement des dimensions respectives qui sont très petites, de façon à ne pas gêner la vision du porteur et être invisibles. En outre, les pistes 40-48 peuvent être constituées de tout matériau qui est à la fois transparent et conducteur électriquement, tel que l'oxyde d'indium dopé à l'étain, ou ITO pour «indium-tin oxide» en anglais. Alternativement, chaque piste 40-48 peut être remplacée par un fil conducteur très fin, qui est disposé radialement dans la partie périphérique du verre. Sur la figure 4a, C désigne un contour de détourage qui est indiqué à titre d'exemple. Le contour C coupe chacune des pistes 40-48 dans une partie de celle-ci qui est dirigée radialement.

Selon un deuxième type de réalisations de l'invention, chaque source de rayonnement peut être située à l'extérieur du verre correspondant, et être couplée optiquement à une des sections de sortie par un premier guide optique, par exemple par une fibre optique. De même, chaque détecteur de rayonnement peut aussi être situé à l'extérieur du verre, et aussi être couplé optiquement à une des sections d'entrée de rayonnement par un second guide optique, qui peut être distinct ou confondu avec l'un des premiers guides optiques. Selon un mode de fabrication particulier du verre, chaque premier ou second guide optique peut être incorporé au verre lors du moulage de celui-ci, en étant disposé dans un moule d'injection avant que le matériau constitutif du verre soit introduit dans le moule. Eventuellement, chaque section de sortie ou d'entrée du rayonnement peut être pourvue d'un micro-prisme qui est intégré au verre, et qui détermine la direction d'émission du rayonnement vers une des zones oculaires, ou la direction de collecte de la partie réfléchie du rayonnement. Autrement dit, chaque section de sortie ou d'entrée du rayonnement est formée par une face d'un microprisme situé à l'extrémité d'un guide optique. Sur la figure 5, les références 50-53 et 60-63 désignent des fibres optiques qui relient respectivement les sections de sortie et d'entrée 10-13 et 20-23. Pour la même raison que précédemment à propos des pistes conductrices, les fibres optiques 50-53 et 60-63 sont de préférence disposées radialement dans le verre 1, pour que leurs positions au niveau du contour de détourage C soient facilement connues.

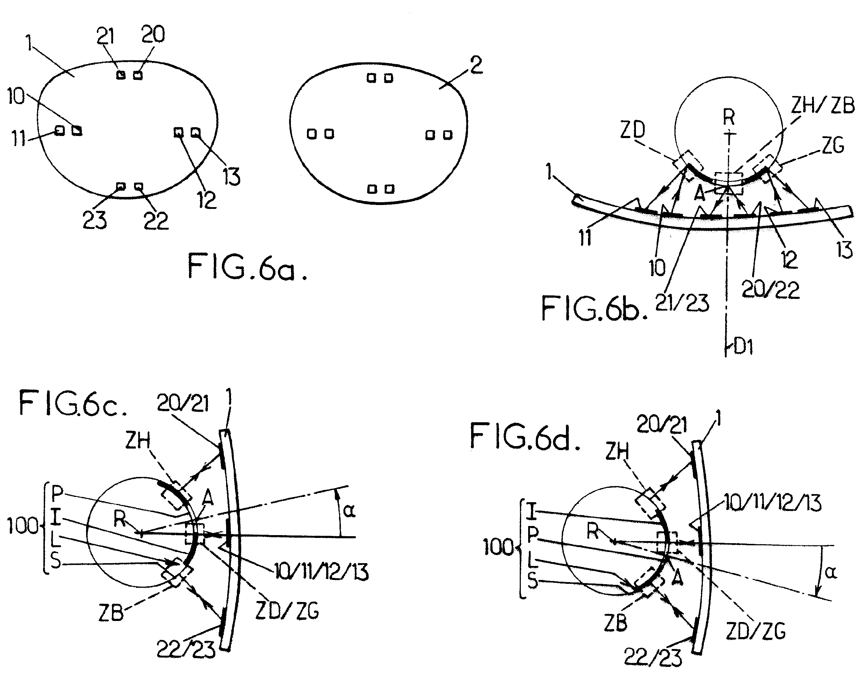

Les figures 6a à 6d illustrent des seconds modes de réalisation d'un verre selon l'invention, dans lequel les sections de sortie et d'entrée de rayonnement déterminent une première paire de chemins optiques dont les points de réflexion sur l'oeil sont décalés en excentricité, et une seconde paire de chemins optiques dont les points de réflexion sur l'oeil sont décalés en hauteur, pour chaque verre de la paire de lunettes. Un tel second mode de réalisation peut être déduit des premiers modes de réalisation des figures 2a à 2d, en redisposant les sections de sortie de rayonnement 20 et 22 selon la direction verticale, correspondant à une valeur nulle de l'excentricité β. Les sections d'entrée de rayonnement 21 et 23 sont redisposées simultanément, pour rester juxtaposées respectivement aux sections de sortie de rayonnement 20 et 22. Ainsi, la section de sortie 10 et la section d'entrée 11 forment un premier couple d'émission-réception, avec un point de réflexion du rayonnement qui est situé dans une zone ZD où se déplace une portion droite du limbe L. La section de sortie 12 et la section d'entrée 13 forment un deuxième couple d'émission-réception, avec un point de réflexion du rayonnement qui est situé dans une zone ZG où se déplace une portion gauche du limbe L. Les sections de sortie et d'entrée de rayonnement 10 à 13 peuvent être disposées pour que les points de réflexion des chemins correspondants du rayonnement aient une valeur de la hauteur α qui est nulle. Les valeurs d'excentricité β des points de réflexion de ces premier et deuxième couples d'émission-réception peuvent être de +/- 25°, par exemple. Simultanément, la section de sortie 20 et la section d'entrée 21 forment un troisième couple d'émission-réception, avec un point de réflexion du rayonnement qui est situé dans une zone ZH où se déplace une portion supérieure du limbe L. La section de sortie 22 et la section d'entrée 23 forment un quatrième couple d'émission-réception, avec un point de réflexion du rayonnement qui est situé dans une zone ZB où se déplace une portion inférieure du limbe L. La figure 6b est une vue en plan qui correspond à la figure 2b, en montrant les zones oculaires ZD et ZG d'un côté et de l'autre du verre 1, et les zones oculaires ZH et ZB qui apparaissent superposées au droit du centre du verre. Les figures 6c et 6d sont des vues de l'oeil 100 situé derrière le verre 1, dans un plan vertical qui passe par le centre de rotation R, respectivement lorsque cet oeil est tourné vers le haut et vers le bas. Ainsi, les figures 6c et 6d illustrent deux valeurs qui ont des signes opposés pour la hauteur α de l'axe optique D1. Les couples d'émission-réception 20/21 et 22/23 permettent alors de déterminer la valeur de la hauteur α de l'axe optique D1, de la même façon que les couples d'émission-réception 10/11 et 12/13 permettent de déterminer la valeur de l'excentricité β du même axe optique D1, et qui a déjà été décrite. Un mode de réalisation de l'invention qui est conforme aux figures 6a à 6d permet donc de déterminer séparément et symétriquement les valeurs respectives de la hauteur α et de l'excentricité β de chaque axe optique D1, D2, et donc de déterminer symétriquement les valeurs respectives de la hauteur et de l'azimut de la direction de regard D0.

Enfin, la figure 7 illustre encore un autre mode de réalisation de l'invention, qui est déduit de celui des figures 6a à 6d. Les sections de sortie de rayonnement et les sections d'entrée de rayonnement sont remplacées dans les parties droite et gauche de chaque verre de lunettes, respectivement par des colonnes de sections de sortie et des colonnes de sections d'entrée. Ainsi, la partie droite du verre 1 contient une colonne de sections de sortie 10₁,..., 10ₙ, où n est un entier naturel supérieur à 2, et une colonne de sections d'entrée 11₁,..., 11ₙ. Ces deux colonnes sont verticales par rapport à la position d'utilisation du verre 1 par le porteur, parallèles, de préférence rapprochées l'une de l'autre, avec les sections de sortie et d'entrée qui sont décalées progressivement vers le bas du verre avec des décréments de hauteur qui sont identiques. Toutefois, il n'est pas nécessaire que les sections à l'intérieur d'une même colonne soient contigües, de sorte qu'elles peuvent être séparées deux à deux par des intervalles. La partie gauche du verre 1 contient une colonne de sections de sortie 12₁,..., 12ₙ et une colonne de sections d'entrée 13₁,..., 13ₙ qui peuvent être analogues à celles de la partie droite du même verre 1. Le verre 2 possède alors une structure symétrique de celle du verre 1. Eventuellement, ces colonnes de sections de sortie ou d'entrée peuvent être constituées directement par des colonnes de sources, par exemple du type diode à émission lumineuse infrarouge, ou par des colonnes de détecteurs, par exemple du type photodiode ou phototransistor.

L'utilisation de telles colonnes de sources ou de détecteurs est particulièrement avantageuse pour obtenir une caractérisation de la direction de regard du porteur, sans nécessiter une consommation d'énergie qui soit trop importante. La source d'énergie qui alimente les sources, les détecteurs et l'unité de calcul peut alors être de capacité réduite. En effet, les sources et détecteurs qui correspondent à des chemins de rayonnement ayant des réflexions sur l'oeil qui sont situées à une même hauteur de zone oculaire, peuvent être sélectionnées conformément à l'une des stratégies suivantes, alors que les autres sources et détecteurs ne sont pas activés :
/i/ les sources et détecteurs peuvent être activés de façon séquentielle, selon un ordre de balayage qui est prédéterminé ;
/ii/ les sources et détecteurs qui sont activés pour une nouvelle séquence de mesure d'intensités de parties de rayonnement réfléchies sur l'oeil peuvent être sélectionnés en fonction d'au moins une valeur d'un rapport signal/bruit qui a été obtenue lors d'une séquence de mesure antérieure ;
/iii/ ils peuvent être sélectionnés en fonction d'un algorithme prédictif, quant à la hauteur de réflexion sur l'oeil qui est la plus adaptée pour caractériser de nouveau la direction du regard du porteur, en fonction des hauteurs qui ont déjà été sélectionnées lors de caractérisations antérieures. Cette stratégie peut tenir compte d'une hauteur de la direction de regard qui a été déterminée antérieurement ; et
/iv/ une stratégie composite qui combine les critères de sélection des stratégies /i/ à /iii/ précédentes.

Ces stratégies sont particulièrement adaptées pour éviter que les mouvements de paupières du porteur ne perturbent la caractérisation de la convergence de son regard, réalisée selon l'invention.

Dans le mode de réalisation qui est illustré par la figure 7, les couples d'émission-réception formés par la section de sortie de rayonnement 20 avec la section d'entrée de rayonnement 21 d'une part, et par la section de sortie de rayonnement 22 avec la section d'entrée de rayonnement 23 d'autre part, sont facultatifs. Par ailleurs, chacun de ces couples d'émission-réception qui est situé dans la partie supérieure ou la partie inférieure du verre, peut aussi être remplacé par une multiplicité de couples qui sont alignés horizontalement, par rapport à la position d'utilisation du verre. Ainsi, une ligne de sources et une ligne de détecteurs peuvent être disposées dans la partie supérieure du verre, horizontalement, avec chaque source de la ligne de sources qui forme un couple d'émission-réception séparé avec le détecteur de la ligne de détecteurs qui est juxtaposée avec cette source. Une même disposition de ligne de sources et de ligne de détecteurs peut aussi être utilisée dans la partie inférieure de chaque verre 1, 2, à la place de la section de sortie 22 et de la section d'entrée 23.

L'Homme du métier comprendra que l'invention n'est pas limitée aux modes de réalisation particuliers qui ont été décrits en relation avec les figures. Notamment, les différents types de réalisations de l'invention qui ont été énumérés peuvent être combinés de multiples façons avec des positions et des tracés différents des chemins de rayonnement, ces chemins comportant des points de réflexion respectifs dans les zones de l'oeil où se déplacent des portions différentes du limbe. En outre, les positions des sections de sortie et d'entrée du rayonnement à l'intérieur de chaque verre peuvent être modifiées librement, pour former des chemins de rayonnement qui comprennent des points de réflexion sur l'oeil situés à l'intérieur de zones variables.

Notamment, les positions relatives de la section de sortie et de la section d'entrée qui appartiennent à un même couple d'émission-réception, en étant juxtaposées l'une avec l'autre, peuvent être modifiées en conservant un point de réflexion du chemin de rayonnement correspondant qui reste au même endroit sur l'oeil. Notamment, la position de la section de sortie et celle de la section d'entrée peuvent être échangées.

Divers perfectionnements de l'invention sont maintenant décrits, qui augmentent la fiabilité de la détermination de la direction de regard, et/ou qui améliorent le confort et/ou la sécurité du porteur de la paire de lunettes.

Selon un premier de ces perfectionnements, la paire de lunettes peut comprendre en outre des moyens de modulation du rayonnement qui est produit par chaque source, et des moyens de traitement du signal de détection qui est produit par chaque détecteur. Ces moyens sont adaptés pour réaliser une détection synchrone de chaque partie du rayonnement qui est réfléchie sur l'oeil du porteur, basée sur la modulation de la source. Une telle détection synchrone permet en particulier de distinguer le rayonnement qui est mis en oeuvre pour l'invention d'un rayonnement ambiant parasite. En particulier, la modulation peut être sélectionnée pour que la détection synchrone supprime efficacement des contributions du rayonnement ambiant à des fréquences qui sont multiples de 50 Hz (Hertz), de telles contributions étant couramment produites par des systèmes d'éclairage à décharge.

Selon un deuxième perfectionnement, la paire de lunettes peut comprendre en outre des moyens de filtrage d'un signal de mesure qui est produit par chaque détecteur. Ces moyens peuvent être adaptés pour supprimer des mesures qui correspondent à des variations involontaires de la direction de regard du porteur. En effet, les variations involontaires de la direction de regard sont en général beaucoup plus rapides que les variations qui résultent de changements volontaires entre des objets qui sont regardés successivement par le porteur. De cette façon, la détermination de la direction de regard du porteur peut être limitée à des changements d'attention visuelle dont le porteur est conscient. Ainsi, lorsque l'invention est utilisée pour commander une caractéristique variable des verres, cette caractéristique n'est modifiée que dans une mesure utile par rapport à l'attention visuelle du porteur.

A l'inverse, selon un troisième perfectionnement de l'invention, les moyens de filtrage peuvent être adaptés pour sélectionner des mesures qui correspondent à des variations involontaires de la direction de regard du porteur. Pour cela, les moyens de filtrage peuvent sélectionner les variations rapides des axes optiques des yeux, qui correspondent à des fréquences de variation qui sont supérieures à une valeur limite fixée.

Enfin, selon un quatrième perfectionnement, la paire de lunettes peut comprendre encore des moyens de contrôle de chaque source de rayonnement, qui sont adaptés pour activer un fonctionnement intermittent de cette source conformément à un rapport cyclique compris entre 2% et 50%, de préférence inférieur à 10%. La valeur de ce rapport cyclique peut être sélectionnée en fonction de plusieurs critères différents, parmi lesquels on peut citer une limitation de la quantité de rayonnement qui est dirigée vers l'oeil et une limitation de la puissance électrique qui est consommée par un verre de lunettes selon l'invention. En effet, un tel verre est de préférence alimenté en courant électrique à partir d'une pile ou d'une batterie qui est logée dans la monture. Par ailleurs, la source peut être activée pour effectuer deux déterminations successives de la direction de regard du porteur qui sont séparées par une durée fixée. Une telle durée d'attente est en général indépendante du rapport cyclique de fonctionnement des sources. Elle peut être déterminée, notamment, en fonction du temps qui est nécessaire à l'unité de calcul pour déterminer la direction de regard à partir des signaux de mesure produits par les détecteurs.

## Revendications

1. Paire de lunettes ophtalmiques, comprenant une monture (3) et deux verres (1, 2) maintenus dans la monture pour être situés respectivement devant les yeux (100, 200) d'un porteur des dites lunettes, et permettant une vision distincte pour le porteur à travers chaque verre, la paire de lunettes comprenant pour chaque verre :
- au moins une source d'un rayonnement sélectionnée pour que ledit rayonnement soit réfléchi par la sclérotique (S) et par l'iris (I) de chaque oeil du porteur conformément à des coefficients d'intensité de réflexion différents respectivement pour la sclérotique et pour l'iris ;
- au moins un détecteur disposé pour mesurer des intensités respectives de parties du rayonnement produit par la source, réfléchies respectivement dans des zones oculaires dans chacune desquelles se déplace une portion différente d'une limite (L) entre la sclérotique et l'iris de l'oeil situé derrière ledit verre lors de mouvements oculaires ; et
- au moins quatre sections de sortie ou d'entrée de rayonnement disposées dans le verre, chaque section de sortie (10, 12, 20, 22) étant disposée pour diriger une partie du rayonnement produit par la source vers au moins une des dites zones oculaires, et chaque section d'entrée (11, 13, 21, 23) étant disposée pour collecter une partie du rayonnement réfléchie dans l'une des dites zones oculaires ;
de façon à former au moins deux chemins de rayonnement comportant chacun une réflexion sur l'oeil du porteur situé derrière ledit verre, dans l'une des zones oculaires où se déplacent les portions de la limite entre la sclérotique et l'iris dudit oeil ;
la paire de lunettes comprenant en outre des moyens pour séparer les parties de rayonnement transmises entre ladite au moins une source de rayonnement et ledit au moins un détecteur respectivement par des chemins de rayonnement différents ; et
la paire de lunettes comprenant en outre une unité de calcul adaptée pour caractériser une direction de regard du porteur vers un point d'observation (C), en fonction des intensités mesurées simultanément pour les deux verres,
la paire de lunettes étant **caractérisée en ce qu'**elle comprend autant de sections de sortie de rayonnement (10, 12, 20, 22) que de sections d'entrée de rayonnement (11, 13, 21, 23) pour chaque verre, chacune des dites sections de sortie étant juxtaposée dans le verre avec l'une des dites sections d'entrée pour former un couple séparé d'émission-réception de rayonnement correspondant à un des dits chemins de rayonnement, chaque couple d'émission-réception étant disposé devant une des zones oculaires où se déplace l'une des portions de la limite (L) entre la sclérotique (S) et l'iris (I) de l'oeil du porteur situé derrière ledit verre lors des mouvements oculaires.

2. Paire de lunettes selon la revendication 1, dans laquelle, pour chaque verre (1, 2), les dits au moins une source, au moins un détecteur et au moins quatre zones de sortie (10, 12, 20, 22) ou d'entrée (11, 13, 21, 23) de rayonnement sont disposés de façon à former au moins trois chemins distincts pour le rayonnement ayant des réflexions respectives réparties angulairement autour d'une direction centrale d'une orbite de chaque oeil.

3. Paire de lunettes selon la revendication 2, dans laquelle, pour chaque verre (1, 2), les dits au moins une source, au moins un détecteur et au moins quatre zones de sortie (10, 12, 20, 22) ou d'entrée (11, 13, 21, 23) de rayonnement sont disposés de façon à former au moins deux paires de chemins distincts pour le rayonnement, chaque chemin ayant une réflexion dans une zone oculaire (ZD1, ZG1, ZD2, ZG2) où se déplace une portion droite ou gauche de la limite (L) entre la sclérotique et l'iris de l'oeil du porteur situé derrière ledit verre,
et de façon que les dites zones oculaires (ZD1, ZG1, ZD2, ZG2) des chemins de chaque paire soient situées à une même hauteur (α₁, α₂) de zones oculaires associées à ladite paire, et les deux paires de chemins étant associées à des hauteurs respectives distinctes et communes aux deux verres.

4. Paire de lunettes selon la revendication 2 ou 3, dans laquelle deux premières sections de sortie de rayonnement (10, 12) sont disposées dans chaque verre à une même première hauteur (α₁), et deux secondes sections de sortie de rayonnement (20, 22) sont disposées dans chaque verre à une même seconde hauteur (α₂) différente de ladite première hauteur.

5. Paire de lunettes selon l'une quelconque des revendications 2 à 4, dans laquelle, pour chaque verre (1, 2), certaines au moins des sections de sortie de rayonnement sont disposées sous forme de deux colonnes situées respectivement dans des parties droite et gauche dudit verre, chaque section de sortie de la colonne (10₁,..., 10ₙ) située dans la partie droite du verre appartenant à un chemin de rayonnement comportant une réflexion sur l'oeil (100) du porteur dans la zone oculaire où se déplace la portion droite de la limite (L) entre la sclérotique (S) et l'iris (I) dudit oeil, et chaque section de sortie de la colonne (12₁,..., 12ₙ) située dans la partie gauche du verre appartenant à un chemin de rayonnement comportant une réflexion sur l'oeil du porteur dans la zone oculaire où se déplace la portion gauche de ladite limite entre la sclérotique et l'iris de l'oeil.

6. Paire de lunettes selon l'une quelconque des revendications 2 à 5, dans laquelle, pour chaque verre (1, 2), les dits au moins une source, au moins un détecteur et au moins quatre zones de sortie (10, 12, 20, 22) ou d'entrée (11, 13, 21, 23) de rayonnement sont disposés de façon à former au moins deux paires de chemins distincts pour le rayonnement, chaque chemin d'une première des dites paires de chemins ayant une réflexion dans une zone oculaire (ZD, ZG) où se déplace une portion droite ou gauche de la limite (L) entre la sclérotique et l'iris de l'oeil du porteur situé derrière ledit verre, et chaque chemin d'une seconde des dites paires de chemins ayant une réflexion dans une zone oculaire (ZH, ZB) où se déplace une portion supérieure ou inférieure de ladite limite (L) entre la sclérotique et l'iris de l'oeil du porteur.

7. Paire de lunettes selon l'une quelconque des revendications précédentes, dans laquelle l'unité de calcul est adaptée pour déterminer d'abord une position angulaire d'un axe optique (D1, D2) de chaque oeil (100, 200) à partir des intensités mesurées simultanément pour le verre (1, 2) situé devant ledit oeil, et pour des parties de rayonnement réfléchies dans certaines des zones oculaires où se déplacent les portions de la limite (L) entre la sclérotique (S) et l'iris (I) dudit oeil ;
et l'unité de calcul est en outre adaptée pour déterminer ensuite la direction de regard du porteur à partir des positions angulaires respectives des axes optiques des deux yeux déterminées pour un même instant.

8. Paire de lunettes selon l'une quelconque des revendications précédentes, dans laquelle chaque source du rayonnement et chaque détecteur sont des composants micro-optoélectroniques à base de matériau semiconducteur, intégrés dans le verre correspondant, et ledit verre comprend en outre des pistes conductrices transparentes (40-48) reliant électriquement des bornes de chaque détecteur et de chaque source à l'unité de calcul, et dirigées radialement dans une partie périphérique du verre.

9. Paire de lunettes selon l'une quelconque des revendications précédentes, comprenant en outre des moyens de variation d'une caractéristique de l'un au moins des verres, en fonction d'un résultat de la direction de regard du porteur **caractérisée par** l'unité de calcul.

10. Utilisation d'une paire de lunettes selon l'une quelconque des revendications précédentes, pour une application sélectionnée parmi la liste suivante :
- occulter une partie d'un champ de vision d'un sujet atteint de dégénérescence maculaire, dynamiquement en fonction de la direction de regard du sujet ;
- constituer une interface d'entrée de communication pour un sujet tétraplégique, pour permettre au sujet de transmettre des données à un outil de saisie informatique en variant volontairement la direction de regard ;
- caractériser des mouvements oculaires involontaires chez un sujet ;
- effectuer un traitement d'image asservi à la direction de regard du porteur de la paire de lunettes ; et
- présenter des données visuelles au porteur de la paire de lunettes, d'une façon adaptée dynamiquement en fonction de la direction de regard du porteur.

11. Verre de lunettes (1, 2) comprenant :
- au moins une source d'un rayonnement (10, 12, 20, 22), chaque source étant intégrée audit verre et sélectionnée pour que ledit rayonnement soit réfléchi par une sclérotique (S) et par un iris (I) d'un oeil (100, 200) d'un porteur du verre, conformément à des coefficients d'intensité de réflexion différents respectivement pour la sclérotique et pour l'iris ;
- au moins un détecteur dudit rayonnement (11, 13, 21, 23), chaque détecteur étant intégré audit verre et disposé pour mesurer une intensité d'une partie du rayonnement produit par l'une des sources et réfléchie par l'oeil dans une zone oculaire dans laquelle se déplace une portion différente d'une limite (L) entre la sclérotique et l'iris dudit oeil lors de mouvements oculaires ;
- des pistes conductrices transparentes (40-48) intégrées audit verre, reliant électriquement des bornes de chaque détecteur et de chaque source, et dirigées radialement dans une partie périphérique du verre ; et
- au moins quatre sections de sortie ou d'entrée de rayonnement disposées dans le verre, chaque section de sortie (10, 12, 20, 22) étant disposée pour diriger une partie du rayonnement produit par la source vers au moins une des dites zones oculaires, et chaque section d'entrée (11, 13, 21, 23) étant disposée pour collecter une partie du rayonnement réfléchie dans l'une des dites zones oculaires ;
de façon à former au moins deux chemins de rayonnement comportant chacun une réflexion sur l'oeil du porteur situé derrière ledit verre, dans l'une des zones oculaires où se déplacent les portions de la limite entre la sclérotique et l'iris dudit oeil,
le verre de lunettes étant **caractérisé en ce qu'**il comprend autant de sections de sortie de rayonnement (10, 12, 20, 22) que de sections d'entrée de rayonnement (11, 13, 21, 23), chacune des sections de sortie étant juxtaposée dans le verre avec l'une des sections d'entrée pour former un couple séparé d'émission-réception de rayonnement correspondant à un des dits chemins de rayonnement, chaque couple d'émission-réception étant disposé devant une des zones oculaires où se déplace l'une des portions de la limite (L) entre la sclérotique (S) et l'iris (I) de l'oeil du porteur lors des mouvements oculaires.

12. Verre de lunettes selon la revendication 11, dans lequel les dits au moins une source, au moins un détecteur et au moins quatre zones de sortie (10, 12, 20, 22) ou d'entrée (11, 13, 21, 23) de rayonnement sont disposés de façon à former au moins trois chemins distincts pour le rayonnement ayant des réflexions respectives réparties angulairement autour d'une direction centrale d'une orbite de chaque oeil.

13. Verre de lunettes selon la revendication 12, dans laquelle, pour chaque verre (1, 2), certaines au moins des sections de sortie de rayonnement sont disposées sous forme de deux colonnes situées respectivement dans des parties droite et gauche dudit verre, chaque section de sortie de la colonne (10₁,..., 10ₙ) située dans la partie droite du verre appartenant à un chemin de rayonnement comportant une réflexion sur l'oeil (100) du porteur dans la zone oculaire où se déplace la portion droite de la limite (L) entre la sclérotique (S) et l'iris (I) dudit oeil, et chaque section de sortie de la colonne (12₁,..., 12ₙ) située dans la partie gauche du verre appartenant à un chemin de rayonnement comportant une réflexion sur l'oeil du porteur dans la zone oculaire où se déplace la portion gauche de ladite limite entre la sclérotique et l'iris de l'oeil.

14. Verre de lunettes selon la revendication 12 ou 13, dans lequel les dits au moins une source, au moins un détecteur et au moins quatre zones de sortie (10, 12, 20, 22) ou d'entrée (11, 13, 21, 23) de rayonnement sont disposés de façon à former au moins deux paires de chemins distincts pour le rayonnement, chaque chemin d'une première des dites paires de chemins ayant une réflexion dans une zone oculaire (ZD, ZG) où se déplace une portion droite ou gauche de la limite (L) entre la sclérotique et l'iris de l'oeil du porteur situé derrière ledit verre, et chaque chemin d'une seconde des dites paires de chemins ayant une réflexion dans une zone oculaire (ZH, ZB) où se déplace une portion supérieure ou inférieure de ladite limite (L) entre la sclérotique et l'iris de l'oeil du porteur.

15. Verre de lunettes selon l'une quelconque des revendications 11 à 14, comprenant en outre des moyens de variation d'une caractéristique dudit verre.

## Patentansprüche

1. Ophthalmische Brille, umfassend eine Fassung (3) und zwei Gläser (1, 2), die in der Fassung gehalten werden, um jeweils vor den Augen (100, 200) eines Trägers der Brille angeordnet zu sein, die für den Träger eine getrennte Sicht durch jedes Glas ermöglicht, wobei die Brille für jedes Glas umfasst:
- mindestens eine Strahlungsquelle, die derart ausgewählt ist, dass die Strahlung von der Sklera (S) und der Iris (I) jedes Auges des Trägers gemäß unterschiedlichen Reflexionsstärkekoeffizienten für die Sklera bzw. für die Iris reflektiert wird;
- mindestens einen Detektor, der dazu vorgesehen ist, jeweilige Stärken von Teilen der von der Quelle erzeugten Strahlung, die in Okularzonen reflektiert werden, in denen sich jeweils ein unterschiedlicher Abschnitt einer Grenze (L) zwischen der Sklera und der Iris des Auges, das sich hinter dem Glas befindet, bei Okularbewegungen bewegt, zu messen; und
- mindestens vier Austritts- oder Eintrittsabschnitte von Strahlung, die in dem Glas angeordnet sind, wobei jeder Austrittsabschnitt (10, 12, 20, 22) dazu vorgesehen ist, einen Teil der von der Quelle erzeugten Strahlung zu mindestens einer der Okularzonen zu lenken, und wobei jeder Eintrittsabschnitt (11, 13, 21, 23) dazu vorgesehen ist, einen Teil der in einer der Okularzonen reflektierten Strahlung zu sammeln;
um mindestens zwei Strahlungswege zu bilden, jeweils umfassend eine Reflexion auf dem Auge des Trägers, das sich hinter dem Glas befindet, in einer der Okularzonen, wo sich die Abschnitte der Grenze zwischen der Sklera und der Iris des Auges bewegen;
wobei die Brille ferner Mittel umfasst, um die zwischen der mindestens einen Strahlungsquelle und dem mindestens einen Detektor über jeweils unterschiedliche Strahlungswege übertragenen Strahlungsteile zu trennen; und
wobei die Brille ferner eine Berechnungseinheit umfasst, die dazu vorgesehen ist, eine Blickrichtung des Trägers zu einem Beobachtungspunkt (C) in Abhängigkeit von den gleichzeitig für die beiden Gläser gemessenen Stärken zu charakterisieren,
wobei die Brille **dadurch gekennzeichnet ist, dass** sie ebenso viele Strahlungsaustrittsabschnitte (10, 12, 20, 22) wie Strahlungseintrittsabschnitte (11, 13, 21, 23) für jedes Glas umfasst, wobei jeder der Austrittsabschnitte in dem Glas mit einem der Eintrittsabschnitte nebeneinander liegend angeordnet ist, um ein getrenntes Sende-Empfangs-Paar von Strahlung entsprechend einem der Strahlungswege zu bilden, wobei jedes Sende-Empfangs-Paar vor einer der Okularzonen angeordnet ist, in der sich einer der Abschnitte der Grenze (L) zwischen der Sklera (S) und der Iris (I) des Auges des Trägers, das sich hinter dem Glas befindet, bei Okularbewegungen bewegt.

2. Brille nach Anspruch 1, bei der für jedes Glas (1, 2) die mindestens eine Quelle, der mindestens eine Detektor und die mindestens vier Austrittszonen (10, 12, 20, 22) oder Eintrittszonen (11, 13, 21, 23) von Strahlung derart angeordnet sind, dass sie mindestens drei getrennte Wege für die Strahlung bilden, die jeweilige Reflexionen haben, die im Winkel um eine zentrale Richtung eines Orbits jedes Auges verteilt sind.

3. Brille nach Anspruch 2, bei der für jedes Glas (1, 2) die mindestens eine Quelle, der mindestens eine Detektor und die mindestens vier Austrittszonen (10, 12, 20, 22) oder Eintrittszonen (11, 13, 21, 23) von Strahlung derart angeordnet sind, dass sie mindestens zwei getrennte Wege für die Strahlung bilden, wobei jeder Weg eine Reflexion in einer Okularzone (ZD1, ZG1, ZD2, ZG2) hat, in der sich ein rechter oder linker Abschnitt der Grenze (L) zwischen der Sklera und der Iris des Auges des Trägers, das sich hinter dem Glas befindet, bewegt,
und derart, dass die Okularzonen (ZD1, ZG1, ZD2, ZG2) der Wege jedes Paars auf einer selben Höhe (α₁, α₂) von dem Paar zugeordneten Okularzonen angeordnet sind, und die beiden Paare von Wegen jeweiligen unterschiedlichen und gemeinsamen Höhen der beiden Gläser zugeordnet sind.

4. Brille nach Anspruch 2 oder 3, bei der zwei erste Austrittsabschnitte von Strahlung (10, 12) in jedem Glas auf einer ersten Höhe (α₁) angeordnet sind, und zwei zweite Austrittsabschnitte von Strahlung (20, 22) in jedem Glas auf einer selben zweiten Höhe (α₂), die von der ersten Höhe verschieden ist, angeordnet sind.

5. Brille nach einem der Ansprüche 2 bis 4, bei der für jedes Glas (1, 2) mindestens gewisse der Austrittsabschnitte von Strahlung in Form von zwei Säulen angeordnet sind, die sich in einem rechten bzw. linken Teil des Glases befinden, wobei jeder Austrittsabschnitt der Säule (10₁, ... 10ₙ), der sich in dem rechten Teil des Glases befindet, der einem Strahlungsweg angehört, eine Reflexion auf dem Auge (100) des Trägers in der Okularzone, in der sich der rechte Abschnitt der Grenze (L) zwischen der Sklera (S) und der Iris (I) des Auges bewegt, umfasst, und wobei jeder Austrittsabschnitt der Säule (12₁, ... 12ₙ), der sich in dem linken Teil des Glases befindet, der einem Strahlungsweg angehört, eine Reflexion auf dem Auge des Trägers in der Okularzone, in der sich der linke Abschnitt der Grenze zwischen der Sklera und der Iris des Auges bewegt, umfasst.

6. Brille nach einem der Ansprüche 2 bis 5, bei der für jedes Glas (1, 2) die mindestens eine Quelle, der mindestens eine Detektor und die mindestens vier Austrittszonen (10, 12, 20, 22) oder Eintrittszonen (11, 13, 21, 23) von Strahlung derart angeordnet sind, dass sie mindestens zwei Paare von getrennten Wegen für die Strahlung bilden, wobei jeder Weg eines ersten der Wegepaare eine Reflexion in einer Okularzone (ZD, ZG) hat, in der sich ein rechter oder linker Abschnitt der Grenze (L) zwischen der Sklera und der Iris des Auges des Trägers, das sich hinter dem Glas befindet, bewegt, und wobei jeder Weg eines zweiten der Wegepaare eine Reflexion in einer Okularzone (ZH, ZB), in der sich ein oberer oder unterer Abschnitt der Grenze (L) zwischen der Sklera und der Iris des Auges des Trägers bewegt, hat.

7. Brille nach einem der vorhergehenden Ansprüche, bei der die Berechnungseinheit dazu vorgesehen ist, zuerst eine Winkelposition einer optischen Achse (D1, D2) jedes Auges (100, 200) aus den Stärken zu bestimmen, die gleichzeitig für das Glas (1, 2), das sich vor dem Auge befindet, und für Teile von Strahlung, die in gewissen Okularzonen, in denen sich die Abschnitte der Grenze (L) zwischen der Sklera (S) und der Iris (I) des Auges bewegen, reflektiert wurden, gemessen wurden;
und bei der die Berechnungseinheit ferner dazu vorgesehen ist, sodann die Blickrichtung des Trägers aus den jeweiligen Winkelpositionen der optischen Achsen der beiden Augen, die für einen selben Zeitpunkt bestimmt wurden, zu bestimmen.

8. Brille nach einem der vorhergehenden Ansprüche, bei der jede Quelle der Strahlung und jeder Detektor mikro-optoelektronische Komponenten auf Basis eines Halbleitermaterials sind, die in das entsprechende Glas integriert sind, und das Glas ferner transparente Leiterspuren (40-48) umfasst, die Klemmen jedes Detektors und jeder Quelle mit der Berechnungseinheit elektrisch verbinden und radial in einem Umfangsteil des Glases ausgerichtet sind.

9. Brille nach einem der vorhergehenden Ansprüche, ferner umfassend Mittel zur Variation eines Merkmals mindestens eines der Gläser in Abhängigkeit von einem Ergebnis der Blickrichtung des Trägers, die durch die Berechnungseinheit charakterisiert wurde.

10. Verwendung einer Brille nach einem der vorhergehenden Ansprüche, für eine Anwendung, die in der folgenden Liste ausgewählt ist:
- Verdecken eines Teils eines Sichtfeldes eines Patienten, der an einer Makuladegeneration leidet, dynamisch in Abhängigkeit von der Blickrichtung des Patienten;
- Herstellen einer Kommunikations-Eingangsschnittstelle für einen Tetraplegiker, um es dem Patienten zu ermöglichen, Daten an ein informatisches Erfassungswerkzeug zu übertragen, wobei absichtlich die Blickrichtung variiert wird;
- Kennzeichnen der ungewollten Okularbewegungen bei einem Patienten;
- Durchführen einer Bildverarbeitung, die der Blickrichtung des Trägers der Brille unterworfen ist; und
- Darstellen der visuellen Daten für den Träger der Brille auf eine dynamisch in Abhängigkeit von der Blickrichtung des Trägers angepasste Weise.

11. Brillenglas (1, 2), umfassend:
- mindestens eine Strahlungsquelle (10, 12, 20, 22), wobei jede Quelle in das Glas integriert und derart ausgewählt ist, dass die Strahlung von der Sklera (S) und der Iris (I) eines Auges (100, 200) eines Trägers des Glases gemäß unterschiedlichen Reflexionsstärkekoeffizienten für die Sklera bzw. für die Iris reflektiert wird;
- mindestens einen Detektor der Strahlung (11, 13, 21, 23), wobei jeder Detektor in das Glas integriert und dazu vorgesehen ist, eine Stärke eines Teils der von einer der Quellen erzeugten Strahlung, die von dem Auge in einer Okularzone reflektiert wird, in der sich ein unterschiedlicher Abschnitt einer Grenze (L) zwischen der Sklera und der Iris des Auges bei Okularbewegungen bewegt, zu messen;
- transparente Leiterspuren (40-48), die in das Glas integriert sind und Klemmen jedes Detektors und jeder Quelle elektrisch verbinden und radial in einem Umfangsteil des Glases ausgerichtet sind; und
- mindestens vier Austritts- oder Eintrittsabschnitte von Strahlung, die in dem Glas angeordnet sind, wobei jeder Austrittsabschnitt (10, 12, 20, 22) dazu vorgesehen ist, einen Teil der von der Quelle erzeugten Strahlung zu mindestens einer der Okularzonen zu lenken, und wobei jeder Eintrittsabschnitt (11, 13, 21, 23) dazu vorgesehen ist, einen Teil der in einer der Okularzonen reflektierten Strahlung zu sammeln;
um mindestens zwei Strahlungswege zu bilden, jeweils umfassend eine Reflexion auf dem Auge des Trägers, das sich hinter dem Glas befindet, in einer der Okularzonen, wo sich die Abschnitte der Grenze zwischen der Sklera und der Iris des Auges bewegen;
wobei das Brillenglas **dadurch gekennzeichnet ist, dass** es ebenso viele Strahlungsaustrittsabschnitte (10, 12, 20, 22) wie Strahlungseintrittsabschnitte (11, 13, 21, 23) umfasst, wobei jeder der Austrittsabschnitte in dem Glas mit einem der Eintrittsabschnitte nebeneinander liegend angeordnet ist, um ein getrenntes Sende-Empfangs-Paar von Strahlung entsprechend einem der Strahlungswege zu bilden, wobei jedes Sende-Empfangs-Paar vor einer der Okularzonen angeordnet ist, in der sich einer der Abschnitte der Grenze (L) zwischen der Sklera (S) und der Iris (I) des Auges des Trägers bei Okularbewegungen bewegt.

12. Brillenglas nach Anspruch 11, bei dem die mindestens eine Quelle, der mindestens eine Detektor und die mindestens vier Austrittszonen (10, 12, 20, 22) oder Eintrittszonen (11, 13, 21, 23) von Strahlung derart angeordnet sind, dass sie mindestens drei getrennte Wege für die Strahlung mit jeweiligen Reflexionen, die im Winkel um eine zentrale Richtung eines Orbits jedes Auges verteilt sind, bilden.

13. Brillenglas nach Anspruch 12, bei dem für jedes Glas (1, 2) mindestens gewisse der Austrittsabschnitte von Strahlung in Form von zwei Säulen angeordnet sind, die sich in einem rechten bzw. linken Teil des Glases befinden, wobei jeder Austrittsabschnitt der Säule (10₁, ... 10ₙ), der sich in dem rechten Teil des Glases befindet, der einem Strahlungsweg angehört, eine Reflexion auf dem Auge (100) des Trägers in der Okularzone, in der sich der rechte Abschnitt der Grenze (L) zwischen der Sklera (S) und der Iris (I) des Auges bewegt, umfasst, und wobei jeder Austrittsabschnitt der Säule (12₁, ... 12ₙ), der sich in dem linken Teil des Glases befindet, der einem Strahlungsweg angehört, eine Reflexion auf dem Auge des Trägers in der Okularzone, in der sich der linke Abschnitt der Grenze zwischen der Sklera und der Iris des Auges bewegt, umfasst.

14. Brillenglas nach Anspruch 12 oder 13, bei dem die mindestens eine Quelle, der mindestens eine Detektor und die mindestens vier Austrittszonen (10, 12, 20, 22) oder Eintrittszonen (11, 13, 21, 23) von Strahlung derart angeordnet sind, dass sie mindestens zwei Paare von getrennten Wegen für die Strahlung bilden, wobei jeder Weg eines ersten der Wegepaare eine Reflexion in einer Okularzone (ZD, ZG) hat, in der sich ein rechter oder linker Abschnitt der Grenze (L) zwischen der Sklera und der Iris des Auges des Trägers, das sich hinter dem Glas befindet, bewegt, und wobei jeder Weg eines zweiten der Wegepaare eine Reflexion in einer Okularzone (ZH, ZB), in der sich ein oberer oder unterer Abschnitt der Grenze (L) zwischen der Sklera und der Iris des Auges des Trägers bewegt, hat.

15. Brillenglas nach einem der Ansprüche 11 bis 14, ferner umfassend Mittel zur Variation eines Merkmals des Glases.

## Claims

1. Pair of ophthalmic spectacles, comprising a frame (3) and two eyeglasses (1, 2) held in the frame in order to be located in front of the eyes (100, 200) of a wearer of said spectacles, respectively, and allowing the wearer to see clearly through each eyeglass, the pair of spectacles comprising for each eyeglass:
- at least one radiation source selected so that said radiation is reflected by the sclera (S) and by the iris (I) of each eye of the wearer with reflection intensity coefficients that are different for the sclera and for the iris, respectively;
- at least one detector placed to measure the respective intensities of portions of the radiation produced by the source, which portions are respectively reflected in ocular zones in each of which there moves, during eye movements, a different segment of a limit (L) between the sclera and the iris of the eye located behind said eyeglass; and
- at least four radiation exit or entrance sections placed in the eyeglass, each exit section (10, 12, 20, 22) being placed to direct a portion of the radiation produced by the source toward at least one of said ocular zones, and each entrance section (11, 13, 21, 23) being placed to collect a portion of the radiation, which portion is reflected in one of said ocular zones;
so as to form at least two radiation pathways each including a reflection, in one of the ocular zones in which the segments of the limit between the sclera and the iris of said eye move, from the eye of the wearer located behind said eyeglass;
the pair of spectacles furthermore comprising means for separating the radiation portions transmitted between said at least one radiation source and said at least one detector by different radiation pathways, respectively; and
the pair of spectacles furthermore comprising a processing unit suitable for characterizing a gaze direction of the wearer toward a point of observation (C), depending on the intensities simultaneously measured for the two eyeglasses,
the pair of spectacles being **characterized in that** it comprises as many radiation exit sections (10, 12, 20, 22) as radiation entrance sections (11, 13, 21, 23) for each eyeglass, each of said exit sections being juxtaposed in the eyeglass with one of said entrance sections in order to form a separate radiation emission-reception pair corresponding to one of said radiation pathways, each emission-reception pair being placed in front of one of the ocular zones in which there moves, during eye movements, one of the segments of the limit (L) between the sclera (S) and the iris (I) of the eye of the wearer located behind said eyeglass.

2. Pair of spectacles according to Claim 1, wherein, for each eyeglass (1, 2), said at least one source, at least one detector and at least four radiation exit (10, 12, 20, 22) or entrance (11, 13, 21, 23) zones are placed so as to form at least three separate pathways for the radiation, including respective reflections angularly distributed about a central direction of an orbit of each eye.

3. Pair of spectacles according to Claim 2, wherein, for each eyeglass (1, 2), said at least one source, at least one detector and at least four radiation exit (10, 12, 20, 22) or entrance (11, 13, 21, 23) zones are placed so as to form at least two pairs of separate pathways for the radiation, each pathway including a reflection in an ocular zone (ZD1, ZG1, ZD2, ZG2) in which there moves a right or left segment of the limit (L) between the sclera and the iris of the eye of the wearer located behind said eyeglass,
and so that said ocular zones (ZD1, ZG1, ZD2, ZG2) of the pathways of each pair are located at a given height (α₁, α₂) of ocular zones associated with said pair, and the two pairs of pathways being associated with distinct respective heights that are common to the two eyeglasses.

4. Pair of spectacles according to Claim 2 or 3, wherein two first radiation exit sections (10, 12) are placed in each eyeglass at a given first height (α₁) and two second radiation exit sections (20, 22) are placed in each eyeglass at a given second height (α₂) different from said first height.

5. Pair of spectacles according to any one of Claims 2 to 4, wherein, for each eyeglass (1, 2), certain at least of the radiation exit sections are placed in the form of two columns located in right and left portions of said eyeglass, respectively, each exit section of the column (10₁,..., 10ₙ) located in the right portion of the eyeglass belonging to a radiation pathway including a reflection from the eye (100) of the wearer in the ocular zone in which there moves the right segment of the limit (L) between the sclera (S) and the iris (I) of said eye, and each exit section of the column (12₁,..., 12ₙ) located in the left portion of the eyeglass belonging to a radiation pathway including a reflection from the eye of the wearer in the ocular zone in which there moves the left segment of said limit between the sclera and the iris of the eye.

6. Pair of spectacles according to any one of Claims 2 to 5, wherein, for each eyeglass (1, 2), said at least one source, at least one detector and at least four radiation exit (10, 12, 20, 22) or entrance (11, 13, 21, 23) zones are placed so as to form at least two pairs of separate pathways for the radiation, each pathway of a first of said pairs of pathways including a reflection in an ocular zone (ZD, ZG) in which there moves a right or left segment of the limit (L) between the sclera and the iris of the eye of the wearer located behind said eyeglass, and each pathway of a second of said pairs of pathways including a reflection in an ocular zone (ZH, ZB) in which there moves an upper or lower segment of said limit (L) between the sclera and the iris of the eye of the wearer.

7. Pair of spectacles according to any one of the preceding claims, wherein the processing unit is suitable for firstly determining an angular position of an optical axis (D1, D2) of each eye (100, 200) from intensities measured simultaneously for the eyeglass (1, 2) located in front of said eye, and for radiation portions reflected in certain of the ocular zones in which the segments of the limit (L) between the sclera (S) and the iris (I) of said eye move;
and the processing unit is furthermore suitable for then determining the gaze direction of the wearer from the respective angular positions of the optical axes of the two eyes, which positions are determined at the same instant.

8. Pair of spectacles according to any one of the preceding claims, wherein, each radiation source and each detector are semiconductor-based micro-optoelectronic components integrated into the corresponding eyeglass, and said eyeglass furthermore comprises transparent conductive tracks (40-48) electrically connecting terminals of each detector and of each source to the processing unit, said tracks being radially directed in a peripheral portion of the eyeglass.

9. Pair of spectacles according to any one of the preceding claims, furthermore comprising means for varying a characteristic of at least one of the eyeglasses, depending on a result of the gaze direction of the wearer **characterized by** the processing unit.

10. Use of a pair of spectacles according to any one of the preceding claims, for an application selected from the following list:
- occulting a portion of a field of view of a subject suffering from macular degeneration, dynamically depending on the direction of gaze of the subject;
- constituting an interface for entering into communication with a tetraplegic subject, in order to allow the subject to transmit data to an information-technology input tool by intentionally varying his gaze direction;
- characterizing involuntary eye movements of a subject;
- performing image processing automatically controlled by the gaze direction of the wearer of the pair of spectacles; and
- presenting visual data to the wearer of the pair of spectacles, in a way dynamically adapted depending on the gaze direction of the wearer.

11. Spectacle eyeglass (1, 2) comprising:
- at least one radiation source (10, 12, 20, 22), each source being integrated into said eyeglass and selected so that said radiation is reflected by a sclera (S) and by an iris (I) and an eye (100, 200) of a wearer of the eyeglass, with reflection intensity coefficients that are different for the sclera and for the iris, respectively;
- at least one detector (11, 13, 21, 23) of said radiation, each detector being integrated into said eyeglass and placed to measure an intensity of a portion of the radiation produced by one of the sources and reflected by the eye in an ocular zone in which there moves, during eye movements, a different segment of a limit (L) between the sclera and the iris of said eye;
- transparent conductive tracks (40-48) integrated into said eyeglass, electrically connecting terminals of each detector and of each source, and radially directed in a peripheral portion of the eyeglass; and
- at least four radiation exit or entrance sections placed in the eyeglass, each exit section (10, 12, 20, 22) being placed to direct a portion of the radiation produced by the source toward at least one of said ocular zones, and each entrance section (11, 13, 21, 23) being placed to collect a portion of the radiation, which portion is reflected in one of said ocular zones;
so as to form at least two radiation pathways each including a reflection from the eye of the wearer located behind said eyeglass, in one of the ocular zones in which the segments of the limit between the sclera and the iris of said eye move,
the pair of spectacles being **characterized in that** it comprises as many radiation exit sections (10, 12, 20, 22) as radiation entrance sections (11, 13, 21, 23), each of the exit sections being juxtaposed in the eyeglass with one of the entrance sections in order to form a separate radiation emission-reception pair corresponding to one of said radiation pathways, each emission-reception pair being placed in front of one of the ocular zones in which there moves, during eye movements, one of the segments of the limit (L) between the sclera (S) and the iris (I) of the eye of the wearer.

12. Spectacle eyeglass according to Claim 11, wherein said at least one source, at least one detector and at least four radiation exit (10, 12, 20, 22) or entrance (11, 13, 21, 23) zones are placed so as to form at least three separate pathways for the radiation, including respective reflections angularly distributed about a central direction of an orbit of each eye.

13. Spectacle eyeglass according to Claim 12, wherein, for each eyeglass (1, 2), certain at least of the radiation exit sections are placed in the form of two columns located in right and left portions of said eyeglass, respectively, each exit section of the column (10₁, ... , 10ₙ) located in the right portion of the eyeglass belonging to a radiation pathway including a reflection from the eye (100) of the wearer in the ocular zone in which there moves the right segment of the limit (L) between the sclera (S) and the iris (I) of said eye, and each exit section of the column (12₁, ..., 12ₙ) located in the left portion of the eyeglass belonging to a radiation pathway including a reflection from the eye of the wearer in the ocular zone in which there moves the left segment of said limit between the sclera and the iris of the eye.

14. Spectacle eyeglass according to Claim 12 or 13, wherein said at least one source, at least one detector and at least four radiation exit (10, 12, 20, 22) or entrance (11, 13, 21, 23) zones are placed so as to form at least two pairs of separate pathways for the radiation, each pathway of a first of said pairs of pathways including a reflection in an ocular zone (ZD, ZG) in which there moves a right or left segment of the limit (L) between the sclera and the iris of the eye of the wearer located behind said eyeglass, and each pathway of a second of said pairs of pathways including a reflection in an ocular zone (ZH, ZB) in which there moves an upper or lower segment of said limit (L) between the sclera and the iris of the eye of the wearer.

15. Spectacle eyeglass according to any one of Claims 11 to 14, furthermore comprising means for varying a characteristic of said eyeglass.
